# EUROPÄISCHE PATENTSCHRIFT

(11) **EP 1 575 706 B1**
(45) Veröffentlichungstag und Bekanntmachung des Hinweises auf die Patenterteilung: **23.08.2006**
(21) Anmeldenummer: 04765416.5
(22) Anmeldetag: 20.09.2004
(51) Int. Cl.: B01L 1/00

(54) **KLIMAKAMMER FÜR MIKROSKOPE**
CLIMATIC CHAMBER FOR MICROSCOPES
CHAMBRE CLIMATISEE POUR MICROSCOPES

(30) Priorität: 23.09.2003 DE 10344295; 23.09.2003 DE 10344294
(43) Veröffentlichungstag der Anmeldung: 21.09.2005
(73) Patentinhaber: Evotec Technologies GmbH, 40225 Düsseldorf (DE)
(72) Erfinder: PIRSCH, Matthias, 22299 Hamburg (DE); HUMMEL, Stefan, 25489 Haseldorf (DE)
(74) Vertreter: von Kirschbaum, Alexander
(86) Internationale Anmeldenummer: PCT/EP2004/010531
(87) Internationale Veröffentlichungsnummer: WO 2005/030394

(56) Entgegenhaltungen:
- DE-A- 3 607 575
- US-A- 4 696 902
- PATENT ABSTRACTS OF JAPAN Bd. 2003, Nr. 08, 6. August 2003 (2003-08-06) & JP 2003 107364 A (TOKAI HIT:KK), 9. April 2003 (2003-04-09) in der Anmeldung erwähnt

## Beschreibung

Die Erfindung betrifft eine Klimakammer, die insbesondere für chemische und/oder biologische Proben geeignet ist.

Bei chemischen und/ oder biologischen Proben, wie beispielsweise Zellen, ist es beispielsweise zum Untersuchen des Zellwachstums oder anderer Reaktionen erforderlich, die Probe über einen längeren Zeitraum einem definierten Klima auszusetzen. Insbesondere ist bei dem Klima die Temperatur und der Feuchtigkeitsgehalt innerhalb eines Klimaraumes, in dem die Probe angeordnet ist, von großer Bedeutung. Ferner können Gasbestandteile, wie die Menge an CO₂ etc., relevant sein.

Aus JP 2003107364 ist eine Klimakammer bekannt, innerhalb der ein Probenträger angeordnet ist. Der gebildete Klimaraum umschließt ausschließlich den Probenträger und weist einen transparenten Deckel auf, um mit Hilfe einer Untersuchungsvorrichtung, wie einem Mikroskop, die Probe zu untersuchen bzw. zu beobachten. Das Vorsehen einer derartigen Klimakammer hat den Nachteil, dass die Untersuchungsvorrichtung einen relativ großen Abstand zur Probe aufweisen muss. Ferner erfolgt die Beobachtung der Probe durch einen transparenten Deckel, so dass auf Grund von Brechungen und dgl. optische Signale verfälscht werden können.

Aufgabe der Erfindung ist es, eine Klimakammer zu schaffen, in der die Untersuchungsmöglichkeiten einer Probe verbessert werden.

Die Lösung der Aufgabe erfolgt erfindungsgemäß durch die Merkmale des Anspruchs 1.

Erfindungsgemäß umschließt ein durch ein Gehäuse gebildeter Klimaraum der Klimakammer eine Untersuchungsvorrichtung, wie ein Mikroskop oder dgl., zumindest teilweise. Somit sind insbesondere die wesentlichen Bestandteile einer Untersuchungsvorrichtung, beispielsweise die Optik und die Beleuchtung eines Mikroskops, innerhalb des Klimaraums angeordnet. Insbesondere die Optikeinrichtung oder ein anderer Teil einer Untersuchungseinrichtung kann somit sehr nahe an die zu untersuchende Probe herangeführt werden, ohne dass eine die Messergebnisse verfälschende Außenwand oder ein Deckel einer Klimakammer zwischen der Probe und der Untersuchungsvorrichtung angeordnet sein muss.

Ferner weist die erfindungsgemäße Klimakammer eine Einlassöffnung auf, durch die ein klimatisierender Mediumstrom in den Klimaraum eingeleitet werden kann. Insbesondere handelt es sich hierbei um das Zuführen von Luft einer beispielsweise definierten Luftfeuchtigkeit und/ oder Temperatur. Ggf. kann auch der Bestandteil von Gasen, wie beispielsweise CO₂, definiert sein. Da der Mediumstrom insbesondere eine relativ hohe Luftfeuchtigkeit aufweisen kann, um die Luftfeuchtigkeit in dem Klimaraum zu erhöhen, besteht die Gefahr des Kondensierens an kalten Bauteilen, wie dem Probenträger und insbesondere Teilen der Untersuchungsvorrichtung. Da das Kondensieren an derartigen Bauteilen zu erheblichen Verfälschungen der Untersuchungsergebnisse führen kann, ist der Mediumstrom erfindungsgemäß derart in den Klimaraum eingeleitet, dass zumindest ein Teil des Mediumstroms die Untersuchungsvorrichtung und/ oder den Probenträger anströmt. Vorzugsweise erfolgt sowohl ein Anströmen des Probenträgers als auch der kritischen Bauteile der Untersuchungsvorrichtung. Hierdurch ist ein Kondensieren in diesem Bereich vermieden.

Vorzugsweise weist die Klimakammer eine Ausrichteinrichtung auf, um den Mediumstrom auf die entsprechenden Teile der Untersuchungsvorrichtung und/oder den Probenträger zu richten. Bei der Ausrichteinrichtung kann es sich um eine Seitenwand des Gehäuses handeln, die die Einlassöffnung aufweist und in einem bestimmten Winkel zu den anzuströmenden Teilen ausgerichtet ist. Hierbei ist der Winkel von der Bauart des Mikroskops abhängig. Vorzugsweise beträgt der Anströmwinkel gegenüber dem Probenträger, vorzugsweise gegenüber einer Unterseite des Probenträgers, 30° - 60°, vorzugsweise 40° - 50°. Bei einem in dem Klimaraum horizontal angeordneten Probenträger, bei dem es sich vorzugsweise um eine Mikrotiterplatte handelt, erfolgt das Anströmen von seitlich unten. Vorzugsweise ist die Ausrichteinrichtung verstellund/ oder justierbar. Dies kann durch Vorsehen einer gesonderten Ausrichteinrichtung realisiert werden. Es ist auch möglich, die Lage eines Zuführschlauchs, durch den der Mediumstrom den Klimaraum zugeführt wird, bzgl. der Einlassöffnung zu verschwenken und somit den Anströmwinkel einzustellen. Auch das Vorsehen von Strömungsleitelementen bzw. -blechen u.ä. ist möglich.

Vorzugsweise ist der Mediumstrom derart ausgerichtet, dass 50% - 70% des Mediumstroms die entsprechenden Teile, d.h. insbesondere die Optik der Untersuchungsvorrichtung und den Probenträger, anströmen. Hierdurch kann in bevorzugter Weise einerseits ein Erwärmen der kondensatempfindlichen Bauteile, wie der Linsen, der Beleuchtung des Probenträgers, realisiert werden und somit ein Kondensieren der Feuchtigkeit verhindert werden und zusätzlich ein gleichmäßiges Erwärmen des Klimaraums erzielt werden.

Des Weiteren ist vorzugsweise in der Nähe des Probenträgers ein Temperatursensor angeordnet. Mit Hilfe einer entsprechenden Regelungseinrichtung kann somit die Temperatur im Bereich des Probenträgers sehr genau eingestellt werden. Zusätzlich kann vorzugsweise ebenfalls im Bereich des Probenträgers ein Feuchtigkeitssensor, ein Gassensor etc. vorgesehen sein. Besonders bevorzugt ist es, die Sensoren, insbesondere den Temperatursensor, unterhalb des Probenträgers anzuordnen. Dies ist insbesondere bei Titerplatten vorteilhaft, da somit die Temperatur in der Nähe des Titerplattenbodens und somit annähernd die Temperatur des Titerplattenbodens selbst bestimmt wird. Mit dem Boden kommt die Probe unmittelbar in Kontakt, so dass durch die Anordnung des Temperatursensors nahe und unterhalb des Probenträgers annähernd die Temperatur der Probe bestimmt werden kann. Dies wäre bei dem Anordnen des Temperatursensors oberhalb des Probenträgers nicht in dieser Genauigkeit möglich, da beispielsweise zwischen einem Deckel des Probenträgers und der Probe selbst ein Luftpolster vorhanden ist, das den Wärmeübergang zwischen dem Probenträger und der Probe verschlechtert.

Bei einer besonders bevorzugten Ausführungsform ist die Form des Gehäuses strömungsoptimiert ausgebildet. Dies hat zur Folge, dass sich auf der Gehäuseinnenwand allenfalls eine sehr geringe Menge an Kondensat bildet. Die Strömungsoptimierung kann vorzugsweise dadurch erzielt werden, dass einander benachbarte Wände in einem Winkel von mindestens 90° zueinander angeordnet sind. Bevorzugt ist es, die Gehäusewände in einem Winkel von mehr als 90°, insbesondere von mehr als 120° zueinander anzuordnen. Dies hat zur Folge, dass der Mediumstrom an der Innenseite des Gehäuses entlang gleitet und kaum "tote Ecken" auftreten.

Vorzugsweise weist die Kammerwand der erfindungsgemäßen Klimakammer ein oder mehrere Öffnungen auf, durch die Manipulatoren, beispielsweise Roboterarme, in das Innere der Klimakammer eingeführt werden können.

Vorzugsweise sind zwischen den Manipulatoren und der Kammerwand zur Abdichtung der Öffnung Kragendichtungen oder andere Dichtungen vorgesehen. Sofern die Manipulatoren nicht oder nicht mehr benötigt werden, können diese durch die Öffnungen entfernt bzw. aus der Kammer heraus gezogen werden. Die Öffnungen können sodann beispielsweise mit einem Schraubdeckel oder einem anderen Verschlusselement verschlossen werden. Vorzugsweise werden durch die Öffnungen in der Kammerwand nur die Manipulatoren selbst, d. h. beispielsweise die Roboterarme, in die Kammer eingeführt. Betätigungselemente, Motorenantriebe und dgl. der Manipulatoren sind hierbei vorzugsweise außerhalb der Kammer angeordnet.

Bei einer besonders bevorzugten Ausführungsform der Erfindung ist in dem Bereich, in dem der Probenträger, ein Objektträger oder dgl. angeordnet ist, ein Umlenkelement vorgesehen, durch das ein Umlenken des Medienstroms erfolgt. Hierdurch kann sichergestellt werden, dass beispielsweise bei einem relativ trockenen Medienstrom die Verdunstungsrate einer flüssigen Probe möglichst gering ist.

Um beispielsweise beim Handhaben der Manipulatoren oder auch zur sonstigen Beobachtung die Probe auf einfache Weise beobachten zu können, ist die Kammerwand der Klimakammer zumindest teilweise durchsichtig. Ebenso kann die Kammerwand Fenster aufweisen, durch die ein Beobachten der Probe etc. möglich ist. Hierbei sind die Fenster oder die transparenten Bereiche der Kammerwand vorzugsweise abdunkelbar bzw. verschließbar. Dies kann beispielsweise bei der Untersuchung lichtempfindlicher Proben vorteilhaft sein.

Eine Probe bzw. ein zu untersuchendes Objekt, das in der Klimakammer angeordnet ist, kann ausschließlich durch das Zuführen eines temperierten Medienstroms in die Klimakammer auf die gewünschte Temperatur gebracht werden. Dies gilt ebenso für die Umgebung der Probe bzw. des Objekts oder auch für die gesamte Klimakammer. Zusätzlich oder anstatt der Erwärmung der Probe durch den Mediumstrom kann auch eine Erwärmung durch das Vorsehen von Heizelementen erfolgen. Hierbei kann es sich beispielsweise um unmittelbar in der Klimakammer vorgesehenen elektrische Heizelemente, aber auch um Strahler handeln, die ggf. auch außerhalb der Klimakammer angeordnet sind. Die Erwärmung kann somit auf Grund von Konvektion und/ oder Strahlung erfolgen.

Besonders bevorzugt ist es, dass das Gehäuse zusätzlich eine Auslassöffnung aufweist, so dass das einströmende Medium nicht nur als Leckagemedium den Klimaraum verlässt. Dies hat den Vorteil, dass die Menge an zugeführtem Medium sehr hoch sein kann, ohne Druck innerhalb des Klimaraums aufzubauen. Vorzugsweise ist mit der Auslassöffnung ein Abführkanal, wie ein Schlauch oder dgl., verbunden. Dieser führt vorzugsweise zu einer Klimaerzeugungs- und/ oder -regelvorrichtung, so dass der Mediumstrom in einem geschlossenen System im Kreis gepumpt wird. Mit Hilfe der Klimaregelvorrichtung wird beispielsweise Dampf zur Erhöhung der Luftfeuchtigkeit erzeugt und/ oder der Mediumstrom erwärmt und/ oder Gase eingeleitet. Die Erfindung betrifft somit eine Klimaregeleinrichtung mit einer Klimakammer und einer Klimaregelvorrichtung.

Eine derartige Klimaregelvorrichtung stellt eine selbstständige Erfindung dar gemäß Anspruch 12.

Es ist bekannt, in Klimaregelvorrichtungen, insbesondere Inkubatoren, chemische und/ oder biologische Proben, wie beispielsweise Zellen, über einen längeren Zeitraum hin einem definierten Klima auszusetzen. Hierbei ist es insbesondere erforderlich, die Luftfeuchtigkeit und die Temperatur innerhalb eines vorgegebenen Bereichs zu halten. Zusätzlich ist die Atmosphäre, beispielsweise der CO₂-Gehalt innerhalb des Inkubators, wichtig. Zur Regelung der Feuchtigkeit, insbesondere der Luftfeuchtigkeit, innerhalb eines Inkubators ist es bekannt, einen Schwamm mit Wasser zu beträufeln und mit warmer Luft zu durchströmen. Eine derartige Feuchtigkeitsregelung weist jedoch den Nachteil auf, dass die Zeitspanne vom Einbringen der Probe in die Klimakammer bis zum Erreichen des gewünschten Feuchtigkeitswertes relativ groß ist. Bei einem geforderten Feuchtigkeitswert von 80% und einem Kammervolumen von 50-70 liegt bei derartigen Klimaregelvorrichtungen die Zeitspanne im Bereich von ca. 3-4 min.. Dies hat insbesondere zur Folge, dass es nicht möglich ist, während der Inkubationszeit beispielsweise für Voruntersuchungen, eine Probe aus der Klimakammer zu entnehmen. Dies wäre, um beispielsweise das Zellwachstum über einen gewissen Zeitraum beurteilen zu können, jedoch wünschenswert, da sodann nicht einzelne Proben über unterschiedliche Zeiträume in gesonderten Inkubatoren inkubiert werden müssen, sondern die gesamte Inkubation in einem Inkubator stattfinden kann.

Die erfindungsgemäße Klimaregelvorrichtung weist einen Kanal bzw. einen Raum oder eine Kammer auf, der von dem zu klimatisierenden gasförmigen Medium durchströmt wird. Üblicherweise handelt es sich bei dem Medium um Luft, die ggf. mit Gasen, wie beispielsweise CO₂, angereichert ist. Ferner weist die Klimaregelvorrichtung eine Dampfkammer mit einer Einlassöffnung und einer mit dem Kanal verbundenen Auslassöffnung auf. In der Dampfkammer, die mit einer Dampferzeugungseinrichtung, wie einer Sprühvorrichtung oder einer Heizung, verbunden ist, wird Dampf, beispielsweise Wasserdampf, erzeugt. Ist als Dampferzeugungseinrichtung eine Sprühvorrichtung vorgesehen, wird ein Aerosol, d.h. eine feintröpfige Verteilung der Flüssigkeit, in einem gasförmigen Medium hervorgerufen. Durch eine Heizung wird ein Verdampfen einer Flüssigkeit hervorgerufen. Hierbei ist beim Verdampfen eines Flüssigkeitsbads durch Heizen die Verdampfungsenthalpie bereits enthalten, wohingegen sie beim Versprühen noch über eine Heizeinrichtung zugeführt werden muss.

Erfindungsgemäß ist an der Einlassöffnung und/ oder der Auslassöffnung der Dampfkammer eine Regeleinrichtung angeordnet, um die aus der Dampfkammer in den Kanal gelangende Dampfmenge zu regeln. Erfindungsgemäß wird der Dampf oder ein Aerosol somit nicht unmittelbar in das zu klimatisierende Medium eingebracht, sondern zuvor in einer Dampfkammer gespeichert. Es ist daher möglich, in der Dampfkammer eine Vorratsmenge an Dampf zu erzeugen, die in kurzer Zeit dem zu klimatisierenden Medium zugeführt werden kann. Erfolgt durch die Regeleinrichtung beispielsweise ein vollständiges Öffnen der Ein- und/ oder Austrittsöffnung der Dampfkammer, so dass ein maximaler Dampf-Volumen-Strom aus der Dampfkammer in den Kanal eingleitet wird, so ist es mit der erfindungsgemäßen Klimaregelvorrichtung möglich, in weniger als fünf Minuten, insbesondere in weniger als drei Minuten eine Luftfeuchtigkeit von über 80%, insbesondere über 90% und besonders bevorzugt über 95% zu erzielen. Dies kann beispielsweise bei einem Volumenstrom des gasförmigen Mediums von ca. 40-50 I/Sek., einem Klimakammervolumen von 50-80 I. und einem Volumen der Dampfkammer von ca. 1l. erzielt werden.

Vorzugsweise handelt es sich bei der Dampferzeugungseinrichtung um eine Heizvorrichtung, durch die durch Verdampfung von Wasser bzw. einem Fluid hohe Luftfeuchtigkeit erzeugt wird. Dies hat gegenüber eine Sprüheinrichtung, die ein Aerosol erzeugt, den Vorteil, dass Dampf nicht so leicht auskondensiert bzw. sich an Oberflächen niederschlägt wie Aerosol.

Mit Hilfe der erfindungsgemäßen Regeleinrichtung kann vorzugsweise der Öffnungsquerschnitt der Einlass- und/ oder der Auslassöffnung der Dampfkammer variiert werden. Dies erfolgt vorzugsweise durch ein verschiebbares Abdeckelement, so dass der Öffnungsquerschnitt auf einfache Weise und schnell variiert werden kann. Vorzugsweise handelt es sich bei dem Abdeckelement um einen Proportional-Schieber. Sofern die erfindungsgemäße Klimaregelvorrichtung, insbesondere zur Klimaregelung in Inkubatoren für chemische und/ oder biologische Proben, eingesetzt werden soll, ist die Einlassöffnung der Dampfkammer vorzugsweise mit dem Kanal verbunden. Dies hat den Vorteil, dass durch die Einlassöffnung keine Fremdluft in das System gelangt, durch die Verunreinigungen zugeführt werden könnten.

Bei dem verschiebbaren Abdeckelement kann es sich auch um ein zylinderförmiges Element handeln, das gegenüber einer schlitzförmigen Auslassöffnung verschiebbar ist. Hierbei ist das zylinderförmig Element in entsprechend kreisringförmigen Lagerungen angeordnet, so dass ein leichtgängiges Verschieben möglich ist.

Ebenso ist es möglich, an der Öffnung eine Klappe vorzusehen, so dass die Größe der Austrittsfläche durch die Stellung der Klappe variiert werden kann. Hierbei ist die Form der Klappe vorzugsweise derart ausgebildet, dass der Öffnungswinkel der Klappe zu der Querschnittsfläche direkt proportional ist. Hierdurch ist die Regelung erheblich vereinfacht.

Vorzugsweise weist die Klimaregelvorrichtung eine Filtereinrichtung auf, durch die die in dem zu klimatisierenden Medium enthaltenen Verunreinigungen, Bakterien etc. ausgefiltert werden können. Vorzugsweise ist die Filtereinrichtung in Strömungsrichtung vor der Dampfkammer, insbesondere vor der Auslassöffnung der Dampfkammer, angeordnet. Das Filtern des Mediums erfolgt somit vor der erneuten oder zusätzlichen Anreicherung mit Dampf.

Ferner kann die erfindungsgemäße Klimaregelvorrichtung eine Temperiereinreichung zum Heizen und/ oder Kühlen des klimatisierten Mediums aufweisen. Die Temperiereinrichtung ist vorzugsweise in Strömungsrichtung nach der Dampfkammer angeordnet, so dass das frisch mit Dampf angereicherte Medium anschließend durch die Temperiereinrichtung strömt. Das Vorsehen einer Temperiereinrichtung, durch die ggf. auch ein Kühlen möglich ist, hat den Vorteil, dass die durch das Einbringen des Dampfes hervorgerufene Temperaturerhöhung des Mediums, sofern diese unerwünscht ist, wieder verringert werden kann. Es ist somit möglich, innerhalb großer Bereiche den Feuchtigkeitsgehalt des Mediums unabhängig von der Temperatur des Mediums zu regeln. Der Bereich ist im Wesentlichen nur durch die physikalischen Grenzen, d.h. insbesondere die Fähigkeit der Feuchtigkeitsaufnahme des Mediums in Abhängigkeit der Temperatur begrenzt (Taupunkt). Versuche haben gezeigt, dass das Fluid, das in der Dampfkammer zur Verdampfung vorhanden ist, vorzugsweise auf eine Temperatur von 40 - 65° gebracht wird, da ansonsten der Einfluss auf die Temperatur des zu klimatisierenden Mediums zu hoch ist und eine aufwändige Kühlung erfolgen müsste. Durch das Vorsehen der Kühlung ist es auch möglich, bei einer hohen Feuchtigkeit von vorzugsweise über 90% die Temperatur unter 30° zu halten.

Vorzugsweise ist in der Dampfkammer Flüssigkeit vorgesehen, die von der Heizeinrichtung verdampft wird. Um die Flüssigkeitsmenge innerhalb eines bestimmten vorgegebenen Bereichs zu halten, ist die Dampfkammer vorzugsweise mit einer Flüssigkeitszuführeinrichtung verbunden. Diese kann ggf. einen automatischen Füllstandsmesser aufweisen, so dass ein automatisches Nachfüllen von Flüssigkeit realisiert ist.

Die erfindungsgemäße Klimakammer, die vorzugsweise mit der vorstehend beschriebenen Klimavorrichtung verbunden ist, ist insbesondere auch zur Hautzucht geeignet. Hierbei ist es, wenn die Hautzucht in der erfindungsgemäßen Klimakammer vorgenommen wird, möglich, mit Hilfe des in der Kammer angeordneten Mikroskops während der Zucht Beobachtungen und/oder Untersuchungen durch zuführen. Ebenso kann die Hautzucht oder das Züchten anderer Zellen in einer mit der erfindungsgemäßen Klimaregelvorrichtung verbundenen Klimakammer stattfinden, wobei die Klimakammer kein Mikroskop oder dgl. aufweist.

Nachfolgend wird die Erfindung anhand einer bevorzugten Ausführungsform unter Bezugnahme auf die anliegenden Zeichnungen näher erläutert.

Es zeigen:
- Fig. 1: eine schematische, teilweise geschnittene Seitenansicht der auf ein Mikroskop aufgesetzten Klimakammer,
- Fig. 2: eine schematische, teilweise geschnittene Schnittansicht entlang der Linie II-II in Fig. 1,
- Fig. 3: eine schematische perspektivische, teilweise geschnittenen Ansicht einer Klimaregelvorrichtung und
- Fig.4: eine schematische, geschnittene Seitenansicht einer weiteren Ausführungsform einer in der Klimaregelvorrichtung vorsehbaren Regeleinrichtung.

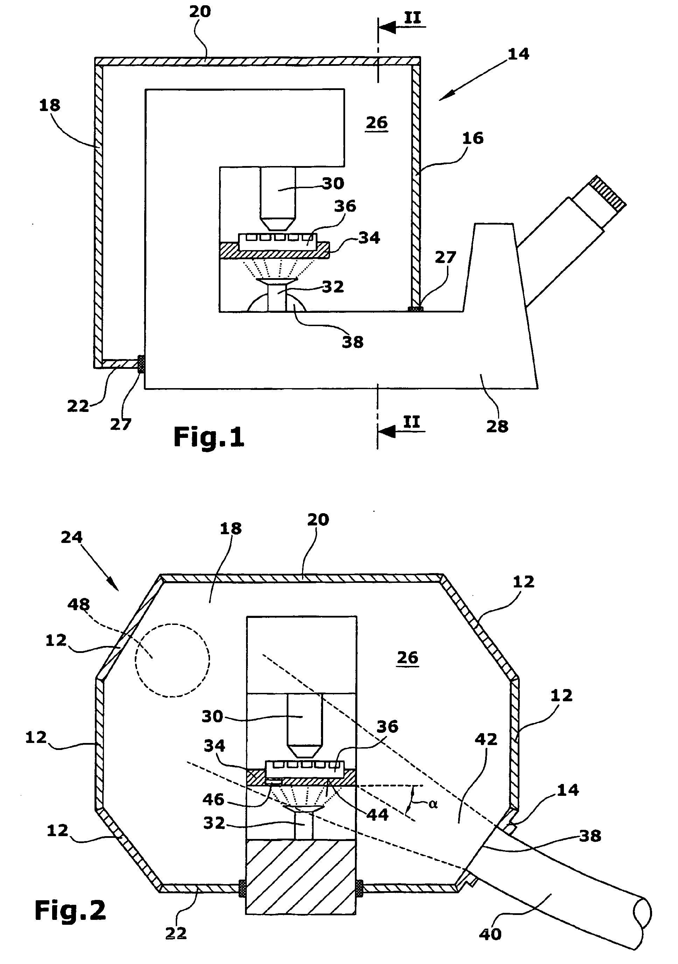

Eine Klimakammer besteht aus mehreren Seitenwänden 12, 14 (Fig. 2), einer Vorderwand 16 (Fig. 1), einer Rückwand 18, einer Deckwand 20 und einer Bodenwand 22. Durch die Wände 12 - 22 ist ein Gehäuse 24 gebildet, dass einen Klimaraum 26 ausbildet. Das Gehäuse 24 weist in der Bodenwand 22 sowie in der Vorderwand 16 eine Ausnehmung auf, so dass das Gehäuse 24 auf ein Mikroskop 28 aufgesetzt werden kann. Die Vorderwand 16 sowie die Bodenwand 22 weisen Dichtelemente 27 auf, die an der Untersuchungsvorrichtung bzw. dem Mikroskop 28 anliegen und den Klimaraum 26 nach außen im Wesentlichen dicht abschließen. Wesentliche Bestandteile der Untersuchungsvorrichtung, bei der es sich im dargestellten Ausführungsbeispiel um ein Mikroskop 28 handelt, sind somit innerhalb des Klimaraums 26 angeordnet. Hierbei handelt es sich insbesondere um eine üblicherweise mehrere Linsen aufweisende Optikeinrichtung 30 sowie eine Beleuchtungseinrichtung 32. Ferner ist ein üblicherweise verschiebbarer Probentisch 34 und ein auf dem Probentisch 34 angeordneter Probenträger 36, bei dem es sich insbesondere um eine Mikrotiterplatte handelt, innerhalb des Klimaraums 26 angeordnet.

Um in dem Klimaraum 26 über einen längeren Zeitraum von mehreren Stunden oder auch mehreren Tagen ein gleichmäßiges Klima erzeugen zu können, weist die Seitenwand 14 eine Einlassöffnung 38 auf, die mit einem Schlauch 40 oder einer anderen Zuführeinrichtung zur Zuführung eines Mediumstroms 42 verbunden ist. Bei dem Mediumstrom 42 handelt es sich vorzugsweise um einen Luftstrom, wobei insbesondere die Feuchtigkeit, die Temperatur und ggf. der Gasgehalt einzelner in dem Mediumstrom vorhandener Gase mit Hilfe einer externen Klimaregeleinrichtung eingestellt wird. Der Mediumstrom 42 ist derart ausgerichtet, dass er bei einem horizontal angeordneten Probenträger 36 seitlich unterhalb des Probenträgers 36 (Fig. 2) angeordnet ist. Ein bevorzugter Anströmwinkel α beträgt hierbei gegenüber dem Probenträger 36 30° - 60°. Innerhalb des Mediumstroms 42 befindet sich neben dem Probenträger 36 auch die Optikeinrichtung 30 sowie die Beleuchtungseinrichtung 32. Insbesondere erfolgt durch den Mediumstrom 42 ein Anströmen einer Unterseite 44 des Probenträgers 36, so dass die innerhalb des Probenträgers angeordnete chemische und/ oder biologische Probe gut temperiert werden kann.

Um die Temperatur im Bereich des Probenträgers 36 und somit annähernd die Temperatur der Probe selbst gut messen zu können, ist unterhalb des Probenträgers 36 ein Temperatursensor 46 angeordnet.

Des Weiteren weist das Gehäuse 24 in der Rückwand 18 eine Auslassöffnung 48 auf. Die Auslassöffnung 48 ist ebenfalls mit einem Schlauch oder dgl. verbunden und leitet den Mediumstrom wieder zurück zu einem Klimaregelgerät, so dass ein Kreislauf des Mediumstroms gewährleistet ist. Die Auslassöffnung 48 ist im Wesentlichen gegenüber der Einlassöffnung 38 angeordnet, um ein möglichst gleichmäßiges Durchströmen des Klimaraums 26 zu gewährleisten. Im Übrigen ist auch die Gehäuseform des Gehäuses 24 möglichst strömungsoptimiert ausgebildet, wobei die in den Zeichnungen dargestellte Ausführungsform vereinfacht dargestellt ist. Insbesondere sind benachbarte Seitenwände vorzugsweise in einem Winkel von mindestens 90°, insbesondere mindestens 120° zueinander angeordnet. Um beispielsweise "tote Ecken" zu vermeiden, könnte zwischen der Rückwand 18 und der Deckwand 20 eine zusätzliche, in Fig. 1 von der Rückwand 18 innerhalb des Klimaraums 26 zur Deckwand 20 verlaufende Wand eingesetzt werden. Hierdurch sind zwischen der Rückwand 18 und der eingesetzten Wand sowie zwischen der Deckwand 20 und der eingesetzten Wand jeweils Winkel von über 90° realisiert. Ferner können die Ecken mit einem Radius versehen werden, um die Bildung von Kondensat in derartigen Ecken zu vermeiden.

Vorzugsweise weist das Gehäuse 24 eine Tür auf, um auf einfache Weise den Probenträger 36 austauschen zu können. Die Tür kann beispielsweise in der Vorderwand 16 vorgesehen und insbesondere auch verschließbar sein.

Die erfindungsgemäße Klimakammer ist insbesondere für konfokale Mikroskope geeignet wobei es sich hierbei um bildergebende oder nicht bildgebende konfokale Mikroskope handeln kann. Ein bildgebendes insbesondere konfokales Mikroskop weist vorzugsweise zur Aufnahme eines Bildes einer Probe ein CCD-Array oder dergleichen auf.

Die erfindungsgemäße Klimaregelvorrichtung weist einen Kanal 110 auf, der in Richtung eines Pfeils 112 von einem zu klimatisierenden, gasförmigen Medium durchströmt wird. Hierzu wird das zu klimatisierende Medium mit Hilfe eines Ventilators 114 durch einen Filter 116, der zur Reinigung des gasförmigen Mediums von Partikeln, Bakterien etc. dient, in den Kanal 110 gesaugt. Nach dem Durchströmen des Mediums durch den Filter 116 und den Kanal 110 wird das Medium durch die Strömungseinrichtung bzw. den Ventilator 114 durch eine Temperiereinrichtung 118, bei der es sich um eine Heiz- und/ oder Kühleinrichtung handelt, geleitet. Das Medium strömt somit durch die Klimaregelvorrichtung in die durch die Pfeile 112 angedeutete Strömungsrichtung.

Unterhalb des Kanals 110 ist eine Dampfkammer 120 vorgesehen. Die Dampfkammer 120 ist innerhalb eines gemeinsamen Gehäuses 122 zusammen mit dem Kanal 110 vorgesehen. Durch eine Trennwand 124 ist der Innenraum des Gehäuses 122 somit in den Kanal 110 und die Dampfkammer 120 unterteilt. Die Dampfkammer 120 ist mit einem zur Dampferzeugung dienenden Heizelement 126 verbunden. Durch das Heizelement 126 wird eine Unterseite 128 des Gehäuses 122 erwärmt. Hierdurch wird das sich in der Dampfkammer befindliche Wasser 130 oder eine andere Flüssigkeit erwärmt, so dass sich oberhalb der Flüssigkeit 130 Dampf 132 bildet. Die Dampfkammer 120 weist eine im dargestellten Ausführungsbeispiel mit dem Kanal 110 verbundene Einlassöffnung 134 auf. Die Einlassöffnung 134 ist in der Trennwand 124 angeordnet. Zusätzlich oder anstatt der Einlassöffnung 134 kann in einem Deckel 136 des Gehäuses 122 eine weitere Einlassöffnung vorgesehen sein. Diese Einlassöffnung ist in dem Bereich unterhalb des Filters 116 in die Seitenwand 136, seitlich neben der Trennwand 124 angeordnet. Hierzu verläuft die Trennwand 124, wie in der Figur dargestellt, nicht durchgehend parallel zur Unterseite 128 des Gehäuses 122, sondern ist auf der einen Seite um etwa 90° abgewinkelt und mit der Seitenwand 136 verbunden.

Ferner weist die Dampfkammer 120 eine mit dem Kanal 110 verbundene Auslassöffnung 138 auf. Durch die Auslassöffnung 138 gelangt Dampf 132 in den Kanal 110, um die Luftfeuchtigkeit des durch die Klimaregelvorrichtung transportierten Mediums zu erhöhen.

Im dargestellten Ausführungsbeispiel ist im Bereich der Auslassöffnung 138 eine Regeleinrichtung 140 in Form eines Schiebers oder eines Abdeckelements vorgesehen. Der Schieber bzw. das Abdeckelement 140 weist eine Öffnung auf, die üblicherweise mindestens die Abmessungen der Auslassöffnung 138 aufweist. Der Schieber 140 ist in Richtung eines Pfeils 142 verschiebbar. Hierdurch ist es möglich, dass die Auslassöffnung 138 und die Öffnung des Schiebers 140 bei maximal geöffneter Regeleinrichtung sich vollständig überdeckend angeordnet sind. Zur Regelung der durch die Auslassöffnung 38 in den Kanal 110 strömenden Dampfmenge, kann der Schieber 140 in Richtung des Pfeils 142 verschoben werden, so dass nur noch ein Teil der Auslassöffnung 138 offen ist. Durch Verschieben des Schiebers 140 in Richtung des Pfeils kann somit der Öffnungsquerschnitt der Auslassöffnung 138 variiert werden.

Um den Flüssigkeitsstand der Flüssigkeit 130 über einen langen Zeitraum konstant halten zu können, kann innerhalb der Dampfkammer 120 ein Füllstandsmesser vorgesehen sein, und die Dampfkammer 120 mit einem Vorratsbehälter verbunden werden.

Zusätzlich ist es möglich, in den Medienstrom über Zuführdüsen, Einlässe oder dgl. Gase, beispielsweise CO₂, einzuleiten.

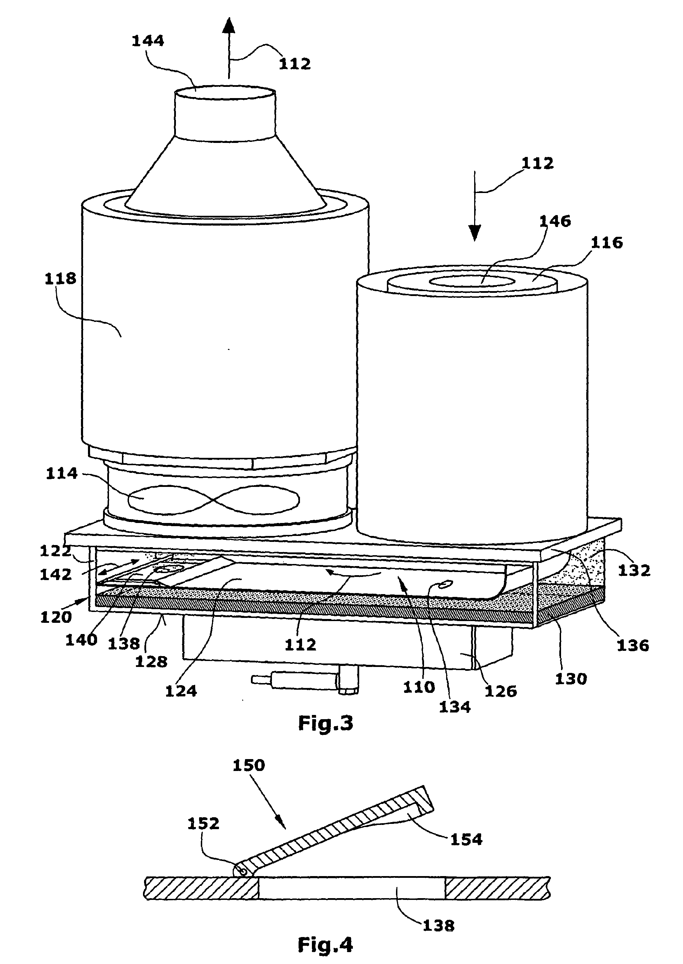

An Stelle des Schiebers 140 (Fig. 3) kann zur Regelung des Öffnungsquerschnitts der Auslassöffnung 138 auch eine Klappe 150 (Fig. 4) vorgesehen sein. Die Klappe 150 ist um eine Achse 152 schwenkbar, wobei je nach Öffnungswinkel der Klappe 150 eine unterschiedliche Menge an Dampf aus der Dampfkammer 120 in die Kammer 110 ausströmen kann. Vorzugsweise weist die Klappe 150 einen in Richtung der Öffnung 138 weisenden Klappenrand 154 auf, der stegförmig ausgebildet ist und eine der Arte der Klappe angepasste Außenkontur aufweist. Durch Wahl bzw. Ausgestaltung der Außenkontur ist es möglich, eine Proportionalität zwischen dem Öffnungswinkel der Klappe 150 und dem hierdurch geöffneten Austrittsquerschnitt der Auslassöffnung 138 zu realisieren, um die Regelung zu vereinfachen.

Bei einer besonders bevorzugten Ausführungsform ist die an Hand der Figuren 1 und 2 beschriebene Klimakammer mit einer an Hand der Figuren 3 und 4 beschriebenen Klimaregelvorrichtung verbunden. Hierzu kann ein Auslass 144 (Fig. 3) mit dem Schlauch 40 (Fig. 2) verbunden werden. Ein an der Auslassöffnung 148 (Fig. 2) vorgesehener Schlauch kann mit einem Einlass 146 des Filters 116 (Fig. 3) verbunden sein. Die beiden Vorrichtungselemente sind somit nur über zwei Schläuche miteinander verbunden und können in einem Abstand zueinander angeordnet werden. Auf Grund dieses modularen Aufbaus ist es möglich, die einzelnen Vorrichtungsbestandteile in unterschiedlichen Ausgestaltungsformen miteinander zu verbinden. Ferner ist die Anordnung in einem Labor vorteilhaft möglich, so dass beispielsweise die Klimaregelvorrichtung (Fig. 3) nicht störend in der Nähe der Klimakammer angeordnet werden muss.

Des weiteren weist die erfindungsgemäße Vorrichtung eine Steuereinrichtung auf. Diese kann mit unterschiedlichen Sensoren, Stelleinrichtungen und dgl. kommunizieren bzw. verbunden sein. Mit Hilfe der Steuereinrichtung und der mit dieser verbundenen Einrichtungen, bei denen es sich beispielsweise um Sensoren handelt, kann das Medium eingestellt werden. Hierbei erfolgt insbesondere ein Einstellen des Feuchtigkeitsgehalts, der Temperatur, des CO₂₋Gehalts, des Gehalts anderer Gasanteile etc.. Ferner kann durch entsprechende Stelleinrichtungen der Anströmwinkel des Medienstroms 42 (Fig. 2) variiert werden. Dies kann durch im Medienstrom vorgesehene Lenkelemente, wie Lenkbleche, die selbstverständlich auch aus Kunststoff sein können, erfolgen oder auch durch Variieren der Lage des Schlauchs 40 bzw. der Einrittsöffnung 38.

## Patentansprüche

1. Klimakammer, insbesondere für chemische und/ oder biologische Proben, mit
einem einen Klimaraum (26) bildenden Gehäuse (24),
einer zumindest teilweise in dem Klimaraum angeordneten Untersuchungsvorrichtung (28) zur Untersuchung der Probe und
einer in dem Gehäuse (24) vorgesehenen Einlassöffnung (38) zum Zuführen eines klimatisierenden Mediumstroms (42),
wobei der Mediumstrom (42) zumindest teilweise die Untersuchungsvorrichtung (28) und/ oder einen in dem Klimaraum (26) angeordneten Probenträger (36) anströmt.

2. Klimakammer nach Anspruch 1, **gekennzeichnet durch** eine Ausrichteinrichtung zum Ausrichten des Mediumstroms (42).

3. Klimakammer nach Anspruch 1 oder 2, **dadurch gekennzeichnet, dass** der Mediumstrom (42) derart ausgerichtet ist, dass der Mediumstrom (42) gegen eine Unterseite (44) des Probenträgers strömt.

4. Klimakammer nach einem der Ansprüche 1 - 3, **dadurch gekennzeichnet, dass** die Einlassöffnung (38) bei horizontal angeordnetem Probenträger (36) seitlich unterhalb des Probenträgers (36) angeordnet ist.

5. Klimakammer nach einem der Ansprüche 1-4, **gekennzeichnet durch** einen Anströmwinkel (α) von 30° - 60° gegenüber dem Probenträger (36).

6. Klimakammer nach einem der Ansprüche 1 - 5, **dadurch gekennzeichnet, dass** der Mediumstrom (42) derart ausgerichtet ist, dass mindestens 50% - 70% des Mediumstroms (42) die Untersuchungsvorrichtung (28) und/oder den Probenträger (36) anströmen.

7. Klimakammer nach einem der Ansprüche 1 - 6, **dadurch gekennzeichnet, dass** kondensatempfindliche Bauteile (30, 32, 34) der Untersuchungsvorrichtung (28) in dem Mediumstrom (42) angeordnet sind.

8. Klimakammer nach einem der Ansprüche 1 - 7, **gekennzeichnet durch** einen nahe des Probenträgers (36), insbesondere nahe der Unterseite (44) des Probenträgers (36) angeordneten Temperatursensor (46).

9. Klimakammer nach einem der Ansprüche 1 - 8, **gekennzeichnet durch** eine im Gehäuse (24) vorgesehene Auslassöffnung (48), die vorzugsweise im Wesentlichen der Einlassöffnung (38) gegenüberliegend angeordnet ist.

10. Klimakammer nach einem der Ansprüche 1 - 9, **dadurch gekennzeichnet, dass** die Gehäuseform strömungsoptimiert ausgebildet ist.

11. Klimakammer nach einem der Ansprüche 1 - 10, **dadurch gekennzeichnet, dass** benachbarte Gehäusewände (12, 14, 16, 18, 20, 22) einen Winkel von mindestens 90°, vorzugsweise mindestens 120° zueinander aufweisen.

12. Klimaregeleinrichtung mit einer Klimakammer nach einem der Ansprüche 1 - 11, wobei mit der Einlassöffnung (38) eine Klimaregelvorrichtung verbunden ist, mit einem von einem zu klimatisierenden gasförmigen Medium durchströmten Kanal (110), einer Dampfkammer (120) mit einer Einlassöffnung (134) und einer mit dem Kanal verbundenen Auslassöffnung (138), einer mit der Dampfkammer (120) verbundenen Dampferzeugungseinrichtung (126) und einer an der Einlassöffnung (134) und/ oder der Auslassöffnung (138) angeordneten Regeleinrichtung (140) zur Regelung der von der Dampfkammer (120) in den Kanal (110) gelangenden Dampfmenge.

13. Klimaregeleinrichtung nach Anspruch 12, **dadurch gekennzeichnet, dass** durch die Regeleinrichtung (140) der Öffnungsquerschnitt der Einlassöffnung (134) und/ oder der Auslassöffnung (138) regelbar ist.

14. Klimaregeleinrichtung nach Anspruch 12 oder 13, **dadurch gekennzeichnet, dass** die Einlassöffnung (134) mit dem Kanal (110) verbunden ist, so dass ein Teil des zu klimatisierenden Mediums in die Dampfkammer (120) strömt.

15. Klimaregeleinrichtung nach einem der Ansprüche 12 - 14, **dadurch gekennzeichnet, dass** die Dampferzeugungseinrichtung (126) eine Heizeinrichtung zum Erwärmen des zu verdampfenden Mediums aufweist.

16. Klimaregeleinrichtung nach einem der Ansprüche 12 - 15, **gekennzeichnet durch** eine Strömungseinrichtung (114) zur Erzeugung des Mediumstroms in dem Kanal (110).

17. Klimaregelvorrichtung nach einem der Ansprüche 12 - 16, **gekennzeichnet durch** eine mit dem Kanal (110) verbundene Filtereinrichtung (116).

18. Klimaregelvorrichtung nach einem der Ansprüche 12 - 17, **gekennzeichnet durch** eine mit dem Kanal (110) verbundene Temperiereinrichtung (118).

## Claims

1. A climate chamber, in particular for chemical and/or biological samples, comprising
a housing (24) defining a climate compartment (26),
an analysis device (28) arranged at least partially in the climate compartment for analyzing the sample, and
an inlet opening (38) provided in the housing (24) for supplying a conditioning medium flow (42),
wherein the medium flow (42) flows at least partially against the analysis device (28) and/or a sample carrier (36) arranged in the climate compartment (26).

2. The climate chamber according to claim 1, **characterized by** a directing device for directing the medium flow (42).

3. The climate chamber according to claim 1 or 2, **characterized in that** the medium flow (42) is directed such that the medium flow (42) flows against a lower side (44) of the sample carrier.

4. The climate chamber according to one of claims 1-3, **characterized in that** the inlet opening (38) is arranged laterally offset below the sample carrier (36) when the sample carrier (36) is horizontally arranged.

5. The climate chamber according to one of claims 1-4, **characterized by** an approach flow angle (α) of 30°-60° relative to the sample carrier (36).

6. The climate chamber according to one of claims 1-5, **characterized in that** the medium flow (42) is directed such that at least 50 %-70 % of the medium flow (42) flows against the analysis device (28) and/or the sample carrier (36).

7. The climate chamber according to one of claims 1-6, **characterized in that** condensate-sensitive components (30,32,34) of the analysis device (28) are located in the medium flow (42).

8. The climate chamber according to one of claims 1-7, **characterized by** a temperature sensor (46) arranged near the sample carrier (36), in particular near the lower side (44) of the sample carrier (36).

9. The climate chamber according to one of claims 1-8, **characterized by** an outlet opening (48) provided in the housing (24), said outlet opening (48) preferably being arranged substantially opposite the inlet opening (38).

10. The climate chamber according to one of claims 1-9, **characterized in that** the housing is configured such that it promotes an optimum flow.

11. The climate chamber according to one of claims 1-10, **characterized in that** adjacent housing walls (12,14,16,18,20,22) are arranged at an angle of at least 90°, preferably at least 120°, relative to each other.

12. A climate control means comprising a climate chamber according to one of claims 1-11, wherein the inlet opening (38) has connected therewith a climate control device; a channel (110) through which flows a gaseous medium which is to be conditioned; a steam chamber (120) having an inlet opening (134) and an outlet opening (138) connected with said channel; a steam generation means (126) connected with said steam chamber (120); and a control means (140) arranged at the inlet opening (134) and/or the outlet opening (138) for controlling the quantity of steam fed from the steam chamber (120) to the channel (110).

13. The climate control means according to claim 12, **characterized in that** the control means (140) is adapted to control the opening cross section of the inlet opening (134) and/or the outlet opening (138).

14. The climate control means according to claim 12 or 13, **characterized in that** the inlet opening (134) is connected with the channel (110) such that a portion of the medium to be conditioned flows into the steam chamber (120).

15. The climate control means according to one of claims 12-14, **characterized in that** the steam generation means (126) comprises a heating means for heating the medium to be evaporated.

16. The climate control means according to one of claims 12-15, **characterized by** a flow-producing means (114) for producing the medium flow in the channel (110).

17. The climate control means according to one of claims 12-16, **characterized by** a filter means (116) connected with the channel (110).

18. The climate control means according to one of claims 12-17, **characterized by** conditioning means (118) connected with the channel (110).

## Revendications

1. Chambre climatisée, en particulier pour échantillons chimiques et/ou biologiques, comportant
un carter (24) formant un espace climatisé (26),
un dispositif d'examen (28) disposé au moins partiellement dans l'espace climatisé pour examiner l'échantillon et
un orifice d'admission (38) prévu dans le bâti (24) pour introduire un flux de milieu de climatisation (42),
le flux de milieu (42) circulant au moins partiellement sur le dispositif d'examen (28) et/ou un support d'échantillons (36) disposé dans l'espace climatisé (26).

2. Chambre climatisée selon la revendication 1, **caractérisée par** un dispositif d'orientation destiné à orienter le flux de milieu (42).

3. Chambre climatisée selon la revendication 1 ou 2, **caractérisée en ce que** le flux de milieu (42) est orienté de manière à être dirigé contre une face inférieure (44) du support d'échantillons.

4. Chambre climatisée selon l'une des revendications 1 à 3, **caractérisée en ce que**, lorsque le support d'échantillon (36) est disposé horizontalement, l'orifice d'admission (38) est disposé latéralement au-dessous du support d'échantillons (36).

5. Chambre climatisée selon l'une des revendications 1 à 4, **caractérisée par** un angle de circulation (α) de 30° à 60° par rapport au support d'échantillons (36).

6. Chambre climatisée selon l'une des revendications 1 à 5, **caractérisée en ce que** le flux de milieu (42) est orienté de telle sorte qu'au moins 50 % à 70 % du flux de milieu (42) circule sur le dispositif d'examen (28) et/ou le support d'échantillons (36).

7. Chambre climatisée selon l'une des revendications 1 à 6, **caractérisée en ce que** des éléments constructifs (30, 32, 34), sensibles au condensat du dispositif d'examen (28) sont disposés dans le flux de milieu (42).

8. Chambre climatisée selon l'une des revendications 1 à 7, **caractérisée par** un capteur de température (46) monté à proximité du support d'échantillons (36), en particulier à proximité de la face inférieure (44) du support d'échantillons (36).

9. Chambre climatisée selon l'une des revendications 1 à 8, **caractérisée par** un orifice d'évacuation (48) prévu dans le carter (24), et situé de préférence sensiblement à l'opposé de l'orifice d'admission (38).

10. Chambre climatisée selon l'une des revendications 1 à 9, **caractérisée en ce que** la forme du carter est conçue pour optimiser la circulation du flux.

11. Chambre climatisée selon l'une des revendications 1 à 10, **caractérisée en ce que** des parois voisines du carter (12, 14, 16, 18, 20, 22) forment un angle d'au moins 90°, de préférence d'au moins 120° l'une par rapport à l'autre.

12. Dispositif de régulation de climatisation comportant une chambre climatisée selon l'une des revendications 1 à 11, où, un dispositif de régulation de climatisation est relié à l'orifice d'admission (38), et comportant un canal (110) où circule un milieu gazeux à climatiser, une chambre à vapeur (120) munie d'un orifice d'admission (134) et un orifice d'évacuation (138) relié au canal, un dispositif (126) de production de vapeur relié à la chambre (120), et un dispositif de régulation (140) associé à l'orifice d'admission (134) et/ou à l'orifice d'évacuation (138) pour réguler la quantité de vapeur acheminée de la chambre à vapeur (120) dans le canal (110).

13. Dispositif de régulation de climatisation selon la revendication 12, **caractérisé en ce que** la section d'ouverture de l'orifice d'admission (134) et/ou de l'orifice d'évacuation (138) est réglable par le dispositif de régulation (140).

14. Dispositif de régulation de climatisation selon la revendication 12 ou 13, **caractérisé en ce que** l'orifice d'admission (134) est relié au canal (110) de sorte qu'une partie du milieu à climatiser circule dans la chambre à vapeur (120).

15. Dispositif de régulation de climatisation selon l'une des revendications 12 à 14, **caractérisé en ce que** le dispositif (126) de production de vapeur présente un dispositif de chauffage pour le réchauffage du milieu à évaporer.

16. Dispositif de régulation de climatisation selon l'une des revendications 12 à 15, **caractérisé par** un dispositif (114) de mise en circulation pour la production du flux de milieu circulant dans le canal (110).

17. Dispositif de régulation de climatisation selon l'une des revendications 12 à 16, **caractérisé par** un dispositif de filtration (116) relié au canal (110).

18. Dispositif de régulation de climatisation selon l'une des revendications 12 à 17, **caractérisé par** un dispositif à tempérer (118) relié au canal (110).
